# EUROPEAN PATENT SPECIFICATION

(11) **EP 3 701 923 B1**
(45) Date of publication and mention of the grant of the patent: **10.01.2024**
(21) Application number: 18895043.0
(22) Date of filing: 17.12.2018
(51) Int. Cl.: A61F 13/531, A61F 13/532, A61F 13/533, A61F 13/495

(54) **ABSORBENT ARTICLE**
ABSORBIERENDER ARTIKEL
ARTICLE ABSORBANT

(30) Priority: 28.12.2017 JP 2017254984
(43) Date of publication of application: 02.09.2020
(73) Proprietor: Unicharm Corporation, Shikokuchuo-shi, Ehime 799-0111 (JP)
(72) Inventor: HASHINO, Yuki, Kanonji-shi, Kagawa 769-1602 (JP); YAMASHITA, Junko, Kanonji-shi, Kagawa 769-1602 (JP); SAKAGUCHI, Satoru, Kanonji-shi, Kagawa 769-1602 (JP)
(74) Representative: Dolleymores
(86) International application number: PCT/JP2018/046296
(87) International publication number: WO 2019/131268

(56) References cited:
- WO-A1-2012/133331
- WO-A1-2013/080837
- WO-A1-2017/212886
- JP-A- 2004 057 413
- JP-A- 2013 208 329
- JP-A- 2014 233 373
- US-A1- 2016 270 971

## Description

### [TECHNICAL FIELD]

The present invention relates to an absorbent article such as a disposable diaper.

### [BACKGROUND ART]

An absorbent article such as a disposable diaper described in Patent Literature 1 includes a front waistline region (ventral side) disposed around the front waistline of a wearer and a rear waistline region (dorsal side) disposed around a rear waistline of the wearer, and a crotch region disposed in the crotch portion of the wearer. An absorbent core that absorbs body fluid of the wearer extends from the front waistline region to the rear waistline region.

In the absorbent article described in the cited document 1, the absorbent core includes a pair of non-laminated portions extending along the front-back direction of the absorbent article. The pair of non-fiber stacking portions are positioned symmetrically relative to a center line passing through the center of the absorbent article in a width direction and extending in the front-back direction. The non-fiber stacking portion extends in the front-back direction from the front waistline region to the rear waistline region. The non-fiber stacking portion is made of a through hole in which no core forming material that forms the absorbent core is present.

### [CITATION LIST]

### [PATENT LITERATURE]

Patent Literature 1: JP 2017-38915 A
WO 2017/212886 A1 discloses an underpants-shaped absorbent article including an absorbent main body having a pair of leak-proof walls each have a joining portion. In one embodiment, the absorbent main body also has a high rigidity region in one or both end portions of the absorbent main body.
WO 2012/133331 A1 relates to an underwear-type disposable diaper having compression parts at specific locations in an absorbent core contained in an absorbent body. It is possible to suppress segmentation of the absorbent core caused by wearer movement.

### [SUMMARY OF INVENTION]

The inventors of the present invention have earnestly studied to find the following problems. Specifically, in a state in which the wearer wears the absorbent article, the crotch region of the absorbent article, particularly the vicinity of the center of the absorbent article in the front-back direction, is sandwiched between the legs of the wearer and deformed so as to be collapsed toward the center in the width direction. Along with this deformation, the region behind the center of the absorbent article in the front-back direction also tends to deform toward the center in the width direction. However, when the region behind the center of the absorbent article in the front-back direction is deformed so as to be collapsed toward the center in the width direction, it is difficult to provide a space (area of the absorbent core) to accommodate feces on the buttock side, thus increasing the possibility of leakage of feces.

Therefore, it is desired to provide an absorbent article capable of more reliably providing a space for storing feces on the buttocks side during wearing.

The present invention provides the absorbent article of independent claim 1. The dependent claims specify preferred but optional features.

An absorbent article according to an embodiment includes a front-back direction, a width direction orthogonal to the front-back direction, a front waistline region, a rear waistline region, a crotch region disposed between the front waistline region and the rear waistline region, and an absorbent core provided at least in the crotch region. A folding line for folding the absorbent article in half extends in the width direction in the crotch region. A high rigidity region is provided behind the folding line and in a region including the center in the width direction. The high rigidity region is defined by a region having rigidity higher than a rigidity in a region between the folding line and the high rigidity region. The length of the high rigidity region in the front-back direction is longer than the length between the folding line and the front edge of the high rigidity region. The absorbent core includes a constricted region constricted inward in the width direction in the crotch region, the constricted region extends in the front-back direction relative to the folding line, and all of the high rigidity region is provided in a region overlapping the constricted region when viewed in the width direction.

### [BRIEF DESCRIPTION OF DRAWINGS]

Fig. 1 is a schematic view of an absorbent article according to an embodiment.
Fig. 2 is an exploded plan view of an absorbent article according to an embodiment.
Fig. 3 is a cross-sectional view of the absorbent article cut along line A-A of Fig. 2.
Fig. 4 is a plan view of an absorbent core of an absorbent article.
Fig. 5 is a schematic view of an absorbent core when receiving force on the inside of a crotch region in a width direction.

### [DESCRIPTION OF EMBODIMENTS]

### (1) Outline of Embodiment

According to the present specification and the accompanying drawings, at least the following matters are disclosed.

An absorbent article according to an embodiment includes a front-back direction, a width direction orthogonal to the front-back direction, a front waistline region, a rear waistline region, a crotch region disposed between the front waistline region and the rear waistline region, and an absorbent core provided at least in the crotch region, in which a folding line for folding the absorbent article in half extends in the width direction in the crotch region, a high rigidity region is provided behind the folding line and in a region including the center in the width direction, the high rigidity region is defined by a region having rigidity higher than a rigidity in a region between the folding line and the high rigidity region, and the length of the high rigidity region in the front-back direction is longer than the length between the folding line and the front edge of the high rigidity region.

In a state in which the wearer wears the absorbent article, the crotch region of the absorbent article, particularly the vicinity of the center of the absorbent article in the the front-back direction, is sandwiched between the legs of the wearer so as to be collapsed toward the center in the width direction. Along with this deformation, the region behind the center of the absorbent article in the front-back direction also tends to deform toward the center in the width direction. This deformation makes it easier for the wearer to move legs during wearing.

In the absorbent article according to this embodiment, the high rigidity region longer in the front-back direction than the length from the folding line to the front edge of the high rigidity region is provided in the region including the center in the width direction behind the folding line. Since the high rigidity region has higher rigidity than the rigidity in the region between the folding line and the high rigidity region, it is difficult to deform the high rigidity region inward in the width direction. Therefore, the absorbent core is difficult to deform inward in the width direction in the high rigidity region and in the rear region therefrom. This facilitates provision of a space for accommodating feces on the buttocks side and decreases leakage of feces.

According to a preferred embodiment, the front edge of the high rigidity region is located 5 to 15 mm behind the folding line.

The position of the folding line of the crotch region is a part strongly pinched by the wearer's leg and this position is easily deformed inward so that the wearer can easily move the leg during wearing. On the other hand, an area behind the position of the folding line 5 to 15 mm behind the folding line is a region opposed to the vicinity of the buttocks of the wearer, and by securing a space for accommodating feces in this area, Leakage can be further decreased.

According to a preferred embodiment, the absorbent core includes a constricted region constricted inward in the width direction in the crotch region, and the high rigidity region is located inside the outer edge of the absorbent core in the width direction for example.

A region inside the constricted region in the width direction is apt to receive a force inward in the width direction by the force received from both legs of the wearer. Since the high rigidity region is provided inside the outer edge of the absorbent core in the width direction, the absorbent core is difficult to deform inward in the width direction in the high rigidity region. This facilitates provision of a space for accommodating feces on the buttocks side and decreases leakage of feces. Furthermore, when the high rigidity region is provided inside the outer edge of the absorbent core in the width direction, it is difficult for the high rigidity region to directly touch the legs of the wearer, so that a sense of discomfort given to the wearer can be suppressed.

According to a preferred embodiment, the length of the high rigidity region in the width direction is shorter than the maximum length of the absorbent core in the width direction.

Accordingly, the area outside the high rigidity region in the width direction is deformed circularly corresponding to the curvature of the wearer's body. In other words, the absorbent core is curved in a cup shape in the width direction. Accordingly, the absorbent core has a shape to accommodate waste matter such as body fluid, and the leakage of body fluid is further decreased.

According to the present invention, the absorbent core includes a constricted region constricted inward in the width direction in the crotch region, the constricted region extends in the front-back direction relative to the folding line, and the high rigidity region is provided in a region overlapping the constricted region when viewed in the width direction.

Since the absorbent core is constricted within the range in which the wearer's legs are movable, the wearer is easy to move the legs. On the other hand, in the region behind the folding line, the high rigidity region is provided in a region overlapping with the constricted region when viewed from the width direction. Accordingly, the absorbent core is less collapsible in the width direction as the absorbent core deviates rearward from the movable range of the legs of the wearer. Accordingly, the area of the absorbent core can be provided more reliably as the absorbent core goes rearward.

According to a preferred embodiment, the high rigidity region is constituted by an embossed region in which at least the absorbent core is compressed in a thickness direction.

Since the embossed area can be embossed to press the absorbent core, the high rigidity region can be formed without increasing the manufacturing cost of the absorbent article. In addition, since the embossed area is a region where the shape of the absorbent core is unlikely to collapse, it is easy to secure the area of the absorbent core.

According to a preferred embodiment, the embossed region is defined by a region including a plurality of dot-shaped embossments recessed from the skin surface side to the non-skin surface side.

Since the embossed area formed in the embossed area is recessed from the skin surface side toward the non-skin surface side, the high rigidity region is easy to bend in a convex shape toward the non-skin surface side. Therefore, the high rigidity region can be deformed in a gentle convex shape toward the non-skin surface side depending on the curvature of the wearer's body, while securing the area of the absorbent core. Accordingly, the absorbent core tends to have a shape to accommodate waste matter such as body fluid, and the leakage of body fluid is further decreased.

According to a preferred embodiment, the absorbent core has a pair of grooves formed on both sides relative to the center of the absorbent article in the width direction, and the pair of grooves extend in the front-back direction relative to the folding line.

The pair of grooves are deformed so as to be closed by being sandwiched between both legs of the wearer, so that the vicinity of the folding line of the crotch region of the absorbent core is easily deformed to be collapsed toward the center in the width direction. This makes it easier for the wearer to move the legs during wearing.

According to a preferred embodiment, at least a part of the high rigidity region is located behind the rear edges of the pair of grooves.

Since the high rigidity region is located behind the pair of grooves which make the absorbent core collapse easily inward in the width direction, it is possible to simultaneously achieve facilitating movement the legs of the wearer in the crotch and reliably providing the area of the absorbent core on the buttocks side.

According to a preferred embodiment, the high rigidity region extends rearward from a region overlapping the pair of grooves when viewed from the width direction than a rear edge of the pair of grooves.

The pair of grooves allows the region in which the fold line is formed in the crotch region to make a portion that is most easily collapsed in the width direction. The high rigidity region allows the region where the high rigidity region overlaps the pair of grooves, when viewed from the width direction, to make a portion hardly collapsed in the width direction than the region where the folding line is formed. Further, the portion extending rearward from the rear edge of the pair of grooves in the high rigidity region includes no pair of grooves and is hardly collapsed in the width direction. As described above, the absorbent core is less collapsible in the width direction as the absorbent core goes rearward from the position of the folding line of the crotch region. Accordingly, the area of the absorbent core can be provided more reliably as the absorbent core goes rearward.

According to a preferred embodiment, the high rigidity region is provided in a region between the pair of grooves in the width direction.

According to this embodiment, by providing a high rigidity region between the pair of grooves serving as starting points for deforming the absorbent core, the entire high rigidity region can be flattened and the area can be further provided.

According to a preferred embodiment, the high rigidity region is adjacent to the pair of grooves in the width direction.

According to this embodiment, the high rigidity region is adjacent to the pair of grooves, the entire region between the pair of grooves can be flattened, and the area of the absorbent core can be provided more reliably.

### (2) Structure of Absorbent Article

An absorbent article according to an embodiment will be described below by referring to the accompanying drawings. In the drawings, the same or similar parts are indicated by the same or similar reference signs. The drawings are illustrated schematically, and dimensional ratio and other variables differ from those of actual measurements. The actual measurements or the like, therefore, should be determined by referring to the following description. The drawings may include different relationships or ratios of measurements.

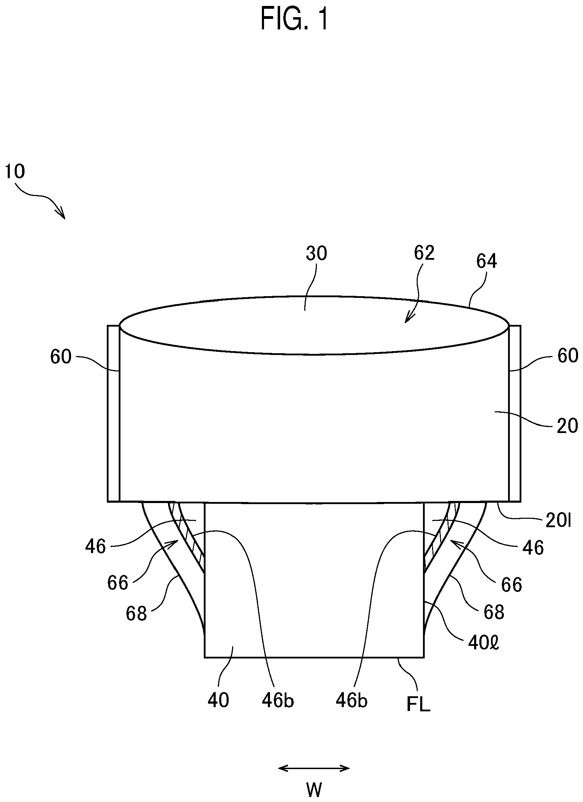

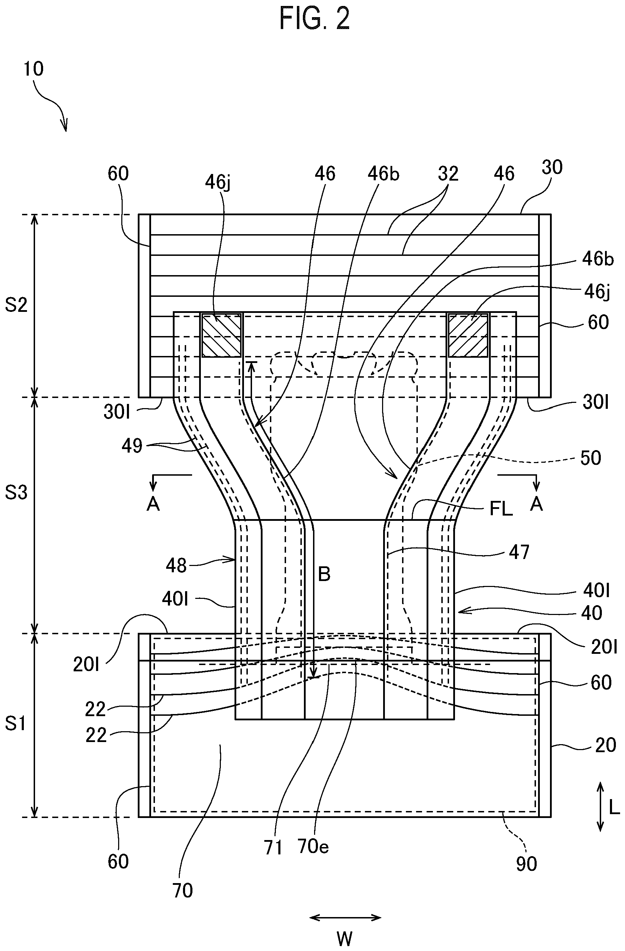

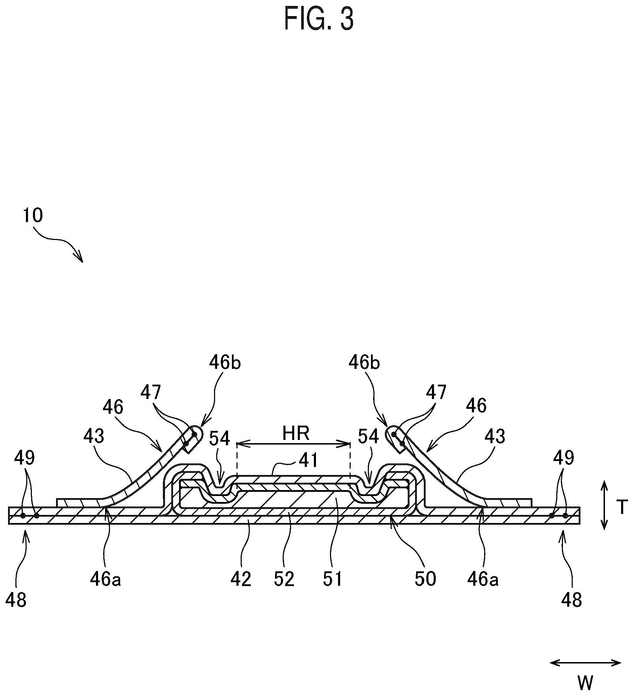

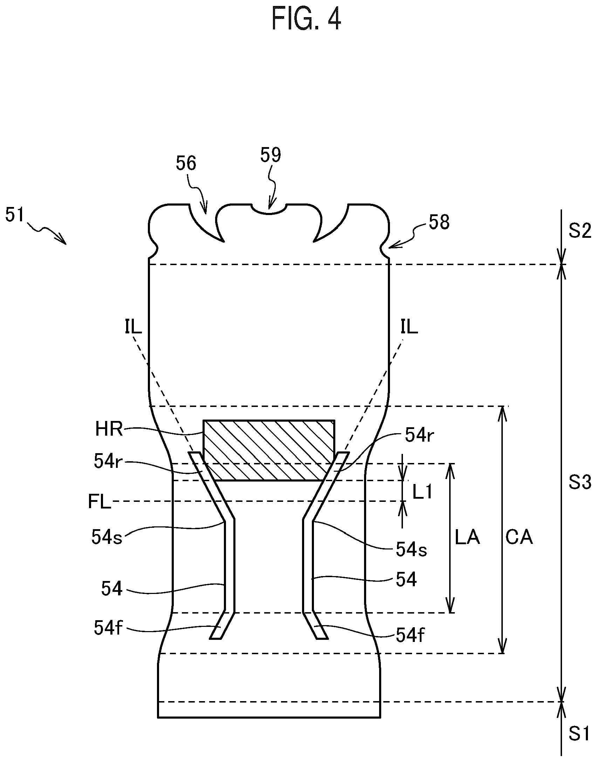

Fig. 1 is a schematic view of an absorbent article according to an embodiment. Fig. 2 is a developed plan view of an absorbent article according to an embodiment. Fig. 2 illustrates an extended state of the absorbent article extended till no wrinkles are formed in a state in which a side engaging portion described later is developed. Fig. 3 is a cross-sectional view of the absorbent article taken along line A-A of Fig. 2. Fig. 4 is a plan view of an absorbent core of an absorbent article.

An absorbent article 10 includes a front-back direction L and a width direction W, both directions being orthogonal to each other in a developed state. The front-back direction L is defined by a direction extending to the front (ventral) side and the rear (dorsal) side of the body. In other words, the front-back direction L is a direction extending forward and backward in the developed absorbent article 10. Further, the absorbent article 10 includes a thickness direction T perpendicular to both the front-back direction L and the width direction W

The absorbent article 10 includes a front waistline region S1, a rear waistline region S2, and a crotch region S3. The front waistline region S1 is a region facing the front waistline of the wearer. The rear waistline region S2 is a region facing the rear waistline of the wearer. The crotch region S3 is a region located in the crotch of the wearer and disposed between the front waistline region S1 and the rear waistline region S2.

In the present embodiment, the absorbent article 10 may include a front waistline member 20, a rear waistline member 30, and an absorbent body 40. The front waistline member 20 is disposed in the front waistline region S1. The rear waistline member 30 is disposed in the rear waistline region S2. The front waistline member 20 and the rear waistline member 30 may be made of a sheet such as a nonwoven fabric.

The front waistline member 20 may include, for example, a non-skin surface side sheet stacked in two layers and a cover sheet 70 stacked in two layers. The non-skin surface side sheet and the cover sheet 70 are made of the integrally-formed sheet. Specifically, the cover sheet 70 is configured by folding the non-skin surface side sheet 14 at the front edge of the front waistline region S1. Alternatively, the non-skin surface side sheet 14 and the cover sheet 70 may be made of different sheets.

In the embodiment illustrated in Fig. 2, a first elastic member 71 extending in the width direction W is provided near a rear edge 70e of the cover sheet 70. Further, the cover sheet 70 may be bonded to the absorbent body 40 with a bonding member 74, e.g., a hot melt adhesive, on the front side of the first elastic member 71.

A folding line FL which bisects the absorbent article 10 into front and rear portions may pass through the crotch region S3. The folding line FL extends in the width direction W. That is, in the state shown in Fig. 1, the absorbent article 10 is folded in half at the folding line FL extending along the width direction W as a base point.

A side engaging portion 60 may be provided at the end portion of the front waistline region S1 in the width direction W and the end portion of the rear waistline region S2 in the width direction W. The side engaging portion 60 is defined by a portion where the end portion of the front waistline region S1 in the width direction W and the end portion of the rear waistline region S2 in the width direction W are engaged with each other.

The end portion of the front waistline region S1 in the width direction W and the end portion of the rear waistline region S2 in the width direction W are locked by the side engaging portion 60. In this state, the absorbent article 10, a waistline opening 62 through which the torso is passed and a pair of leg openings 66 into which the legs of the wearer are inserted. A waistline opening edge 64 forming the waistline opening 62 may be defined by the front edge of the front waistline member 20 and the rear edge of the rear waistline member 30. Further, a leg opening edge 68 forming the leg opening 66 may be defined by a side edge 40l of the absorbent body 40 extending in the front-back direction L, a side 201 located behind the front waistline member 20, and a side 301 located in front of the rear waistline member 30.

Fig. 2 illustrates the state in which the side engaging portion 60 is released and the absorbent article 10 is developed. The side engaging portion 60 may extend in the front-back direction L on the front waistline member 20 and the rear waistline member 30. In this case, the boundary between the front waistline region S1 and the crotch region S3 may be defined by the rear edge of the side engaging portion 60 provided on the front waistline member 20. Similarly, the boundary between the rear waistline region S2 and the crotch region S3 may be defined by the front edge of the side engaging portion 60 provided on the rear waistline member 30.

One or more elastic members 32 that are stretchable in the width direction W may be provided in the rear waistline region S2. Alternatively, the elastic members 32 of the rear waistline region S2 may be made of a stretchable sheet that can expand and contract in the width direction W.

One or more second elastic members 22 that are stretchable in the width direction W may be provided in the front waistline region S1. The second elastic member 22 is made of elastic threads. The second elastic members 22 may be provided on the non-skin surface side of the absorbent body 40 in the front waistline region S1. Further, a stretchable sheet 90 stretchable in the width direction W may be provided in the front waistline region S1. The stretchable sheet 90 may be provided in a region not overlapping the second elastic members 22 in the thickness direction T.

The absorbent body 40 is disposed across the front waistline member 20 and the rear waistline member 30. That is, the absorbent body 40 extends over the front waistline region S1, the rear waistline region S2, and the crotch region S3.

The absorbent body 40 may be formed separately from the front waistline member 20 and the rear waistline member 30. In this case, the absorbent body 40 may be bonded to the front waistline member 20 and the rear waistline member 30, respectively, in the front waistline region S1 and the rear waistline region S2.

The absorbent body 40 at least includes the absorber 50. The absorbent body 40 may also include a skin surface sheet 41 positioned on the skin surface side of the absorber 50 and a non-skin surface sheet 42 positioned on the non-skin surface side of the absorber 50. The skin surface sheet 41 may cover the absorber 50 on the skin surface side of the absorber 50. The non-skin surface sheet 42 may cover the absorber 50 on the non-skin surface side of the absorber 50.

The skin surface sheet 41 may be made of a liquid impermeable sheet, such as a nonwoven fabric or a perforated plastic film. The skin surface sheet 41 may be made of one sheet. Alternatively, the skin surface sheet 41 may be made of a stacked sheet in which a plurality of sheets are stacked.

The non-skin surface sheet 42 may include a liquid impermeable sheet. The liquid impermeable sheet may be made of a liquid impermeable film, such as a plastic film. The non-skin surface sheet 42 may be made of one sheet. Alternatively, the non-skin surface sheet 42 may be made of a laminated sheet in which a plurality of sheets is laminated. In this case, at least one of the plurality of sheets may be liquid impermeable.

The absorber 50 may include an absorbent core 51 and a core wrap 52 enclosing the absorbent core. The absorbent core 51 may include, for example, ground pulp, or superabsorbent polymer (SAP), or mixtures thereof. The core wrap 52 may be made of, for example, a tissue or a nonwoven fabric.

The absorbent core 51 is arranged at least in the crotch region S3. Preferably, the absorbent core 51 may extend from the front waistline region S1 to the rear waistline region S2 in the front-back direction L.

The absorbent core 51 includes a constricted region CA constricted inward in the width direction W at least in the crotch region S3. The constricted region CA extends in the front-back direction L relative to the folding line FL. The narrowest portion LA of the absorbent core 51 in the width direction W may be provided in the crotch region S3. Further, the narrowest portion of the absorbent core 51 may extend in the front-back direction L. In this case, the folding line FW may pass through the constricted region CAof the absorbent core 51.

The absorbent body 40 may include a side sheet 43 covering both side portions of the skin surface sheet 41 in the width direction W. The side sheet 43 may be made of, for example, a sheet, such as a nonwoven fabric or a perforated plastic film.

The absorbent body 40 may include a pair of leakage preventing cuffs 46. The pair of leakage preventing cuffs 46 extends in the front-back direction L between the front waistline region S1 and the rear waistline region S2, and is disposed on both sides of the center of the absorbent article 10 in the width direction W. The pair of leakage preventing cuffs 46 is defined by a portion (a portion of range B in Fig. 2) that can rise toward the skin of the wearer.

The leakage preventing cuffs 46 may be made of the side sheet 43 and a third elastic member 47 provided on the side sheet 43. The third elastic member 47 is configured to be stretchable in the front-back direction. The third elastic member 47 may include one or more rubber threads. In a specific example, the inner side of the side sheet 43 in the width direction W is folded back to the outside, and the third elastic member 47 is disposed in the folded back portion of the side sheet 43. The inner edge of the side sheet 43 in the width direction W is not bonded to the skin surface sheet 41, and forms a free end that can rise from the skin surface sheet 41.

The pair of leakage preventing cuffs 46 each includes a cuff bonding portion 46j bonded in non-rising manner toward the skin surface side on the rear side of the rear edge of each leakage preventing cuff 46. In the illustrated example, the cuff bonding portion 46j is defined by the bonded portion between the side sheet 43 and the skin surface sheet 41. The side sheet 43 and the skin surface sheet 41 may be bonded by an adhesive such as a hot melt adhesive. Although not illustrated, the pair of leakage preventing cuffs 46 may also include the cuff bonding portion in non-rising manner toward the skin surface side on the front side of the front edge of each leakage preventing cuff 46.

Each leakage preventing cuff 46 includes a rising fulcrum 46a and a rising apex 46b. The rising fulcrum 46a and the rising apex 46b extend in the front-back direction L from the front waistline region S1 to the rear waistline region S2. The rising fulcrum 46a is a non-rising point and corresponds to the root of the leakage preventing cuff 46. In the present embodiment, the rising fulcrum 46a corresponds to an edge line on the inner side in the width direction W of the bonding portion between the side sheet 43 and the skin surface sheet 41. The rising apex 46b corresponds to the apex of the leakage preventing cuff 46 when the leakage preventing cuff 46 rises. That is, the rising apex 46b is the free end of the leakage preventing cuff 46.

The absorbent body 40 may include a pair of gathers 48 in a region outside the pair of leakage preventing cuffs 46 in the width direction W. The pair of gathers 48 may extend in the front-back direction L from the front waistline region S1 to the rear waistline region S2.

The pair of gathers 48 may be made of a fourth elastic member 49 attached to a sheet that forms the absorbent body 40. The fourth elastic member 49 may be, for example, one or more rubber threads. Alternatively, the fourth elastic member 49 may be, for example, an elastic sheet having elasticity. The fourth elastic member 49 is bonded to a sheet that forms the absorbent body 40 in a manner stretching from its natural state. Accordingly, the fourth elastic member 49 can form gathers to contract the absorbent body 40 in the front-back direction L.

### (3) Structure of Absorbent Core

Next, the configuration of the absorbent core 51 will be described in detail. Fig. 4 is a plan view of the absorbent core 51 of the absorbent article 10.

The absorbent core 51 may include a pair of rear slits 56 positioned on both sides across the center in the width direction W in the rear waistline region S2. The absorbent core 51 may also include a central slit 59 between the pair of rear slits 56. Further, the absorbent core 51 may include a pair of side slits 58 positioned at both outer edges of the absorbent core 51 in the width direction W. Note that the slits are regions where the absorbent material forming the absorbent core 51 is substantially zero.

The absorbent core 51 may include a pair of grooves 54 formed on both sides relative to the center of the absorbent article 10 in the width direction W. As used herein, the concept of the "groove" of the absorbent core 51 includes a concave portion formed in the absorbent material that forms the absorbent core 51 and a region having a zero basis weight of the absorbent material.

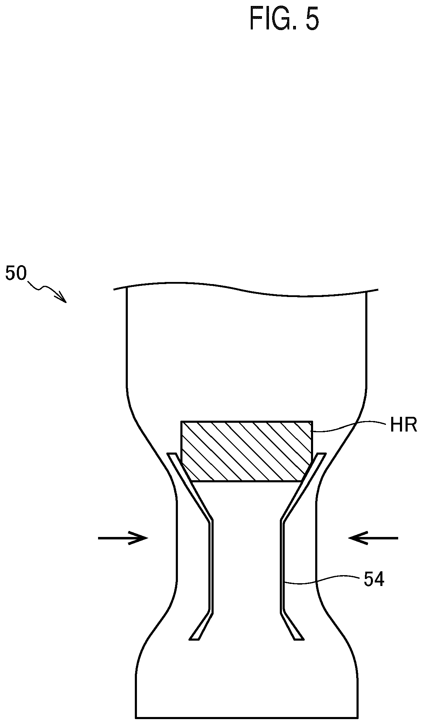

In this embodiment, the constricted region CAof the absorbent core 51 and the pair of grooves 54 cause the folding line FL of the crotch region S3 and the region in front of the folding line FL to be sandwiched between the legs of the wearer and deformed in a collapsing manner toward the center in the width direction W. That is, the absorbent core 51 is easily deformed inward in the width direction W so that the grooves 54 are closed (see Fig. 5). Since the legs of the wearer often move at positions corresponding to the front side of the folding line FL during wearing, the wearer can move the legs easily.

Here, as described above, the narrowest portion LA in which the absorbent core 51 is shortest in the width direction W in the constricted region CA may extend in the front-back direction L. Further, the narrowest portion LA of the absorbent core 51 preferably extends to a position behind the folding line FL. This increases the length of the region where the absorbent core 51 is easily collapsed in the width direction. Therefore, the wearer more easily moves the legs while sandwiching the absorbent core 51 between the legs during wearing.

The absorbent core 51 may include a rearward widening portion 54r in which a distance between the pair of grooves 54 increases as the absorbent core 51 goes rearward. A front edge 54s of the rearward widening portion 54r may be located in a region in front of the folding line FL and overlapping the constricted region CA. More preferably, the front edge 54s of the rearward widening portion 54r may be positioned in a region in front of the folding line FL and overlapping the narrowest portion LA of the constricted region CA. Here, the front edge 54s of the rearward widening portion 54r is defined by the point at which the distance between the pair of grooves 54 starts to go rearward from the region having the narrowest distance between the pair of grooves 54 in the crotch region S3 and at which the distance between the pair of grooves 54 in the crotch region S3 starts to increase.

The front edge 54s of the rearward widening portion 54r is positioned at a region overlapping the constricted region CA. Accordingly, in the rear portion of the constricted region CA, the absorbent core 51 tends to be collapsed inward in the width direction W by the pair of grooves 54, while the width between the pair of grooves 54 can be widened rearward, that is, toward the buttocks side of the wearer. This makes it difficult for the absorbent core 51 to be collapsed inward in the width direction in the rear part of the constricted region CA, so that, in a region where a lot of body fluid is adhered, the area of the absorbent core 51 can be provided reliably in this region.

Meanwhile, a distance between boundaries (flesh slitting lines) of the buttocks and the legs of the wearer increases from the crotch toward the dorsal side of the wearer. The front edge 54s of the rearward widening portion 54r is located in front of the folding line FL, so that the distance between the pair of grooves 54 can increase as the distance between the boundaries (flesh slitting lines) of the buttocks and the leg of the wearer increases. This facilitates provision of the area of the absorbent core 51 on the side of the buttocks conforming to the body shape of the wearer, and decreases leakage of the body fluid. From the viewpoint of facilitating the movement of the legs of the wearer during wearing, the pair of grooves 54 may reach the rear side of the folding line FL.

Preferably, the rear edges of the pair of grooves 54 are located within the range of the constricted region CA in the front-back direction L. That is, the rear side edges of the pair of grooves 54 are located within the range of the constricted region CA, and the pair of grooves 54 does not extend to the rear side from the constricted region CA. This makes it difficult for the absorbent core 51 to collapse inward in the width direction in a region behind the movable range of the legs of the wearer, thus facilitating the provision of the area of the absorbent core 51.

In the illustrated example, the rear side edges of the pair of grooves 54 are located behind the narrowest portion LA of the absorbent core 51. That is, the rear side edges of the pair of grooves 54 are located in the region where the width of the absorbent core 51 is expanded. Accordingly, the region where the absorbent core 51 is less collapsible inward in the width direction gradually increases as the absorbent core 51 goes rearward, thus facilitating provision of the area of the absorbent core 51 as the absorbent core 51 goes rearward from the folding line.

In the crotch region S3, the interval between the pair of grooves 54 may be equal to or larger than 1/4 of the maximum width of the absorbent core 51 in the width direction W. This makes it possible to provide a space between the pair of grooves 54. Accordingly, in the crotch region S3, the minimum area of the absorbent core 51 can also be kept between the pair of grooves 54.

The pair of grooves 54 does not reach the outer edge of the absorbent core 51 in the width direction W. More preferably, the outer edges of the pair of grooves 54 in the width direction W are located inside the narrowest portion LA of the absorbent core 51 in the width direction W. This prevents division of the absorbent core 51 by the pair of grooves 54 and decreases deformation of the absorbent cores 51 disposed adjacent to each other across the grooves 54 from being overlapped each other. Therefore, in the width direction W, the absorbent core 51 tends to be in a cup shape capable of easily holding the body fluid and curved conforming to the body shape.

Preferably, a pair of imaginary lines IL extending the pair of grooves 54 rearward in a direction in which the pair of grooves 54 extends, that is, the rearward widening portion 54r extends, pass the outside of the cuff bonding portion 46j in the width direction W. The imaginary lines IL are lines on which wrinkles tend to be formed and which serve as starting points from which the absorbent core 51 collapses inward in the width direction W. In other words, the wrinkles of the absorbent core 51 tend to be formed on the imaginary lines IL. Since the imaginary lines IL each pass through the outside of the cuff bonding portion 46j in the width direction W, the wrinkles of the absorbent core 51 are hardly formed in the region between the cuff bonding portions 46j. Therefore, the entire region of the absorbent core 51 is hardly collapsed inward in the width direction between the pair of leakage preventing cuffs 46 in the rear end regions of the pair of leakage preventing cuffs 46, so that the area of the absorbent core can be provided easily.

Preferably, the absorbent core 51 includes a forward widening portion 54f in which the distance between the pair of grooves 54 increases toward the front. Since the distance between the pair of grooves 54, which acts as the deformation starting points of the absorbent core 51, also increases on the ventral side, the area of the absorbent core can also be provided easily, and the leakage of the bodily fluid can be further decreased.

The front edges of the pair of grooves 54 are located in the crotch region S3 and do not have to reach the front waistline region S1. Preferably, the front edges of the pair of grooves 54 are located within the constricted region CA of the absorbent core 51.

The absorbent core 51 includes a high rigidity region HR. The high rigidity region HR is provided behind the folding line FL and in a region including the center in the width direction W. The high rigidity region HR is defined by a region having rigidity higher than the rigidity in a region between the folding line FL and the high rigidity region HR. Preferably, the high rigidity region HR may be formed of an embossed region in which the absorbent core 51 is compressed in the thickness direction.

A length of the high rigidity region HR in the front-back direction L is longer than a length L1 between the folding line FL and the front edge of the high rigidity region HR. In a state in which the wearer wears the absorbent article 10, the vicinity of the center of the absorbent article 10 in the crotch region S3 of the absorbent article, particularly in the front-back direction L, is sandwiched between the legs of the wearer and deformed to be collapsed toward the center in the width direction W. Along with this deformation, a region behind the center of the absorbent article 10 in the front-back direction L also tends to deform toward the center in the width direction W. This deformation facilitates movement of the legs of the wearer during wearing (see also Fig. 5). According to the embodiment, the high rigidity region HR which is longer in the front-back direction L than the length L1 between the folding line FL and the front edge of the high rigidity region HR is located at a portion behind the folding line FL and the center in the width direction W. Since the high rigidity region HR has higher rigidity than the rigidity in the region between the folding line FL and the high rigidity region HR, it is difficult to deform inward in the width direction W. Therefore, the absorbent core 51 is hardly deformed inward in the width direction W in the high rigidity region HR and in the region behind the high rigidity region HR. Since the absorbent core 51 is hardly collapsed, on the buttocks side, the area of the absorbent core 51, and the leakage of bodily fluids can be suppressed.

It is preferable that the front edge of the high rigidity region HR is located 5 to 15 mm behind the folding line FL. The position of the folding line FL of the crotch region S3 is a part strongly sandwiched by the wearer's legs and this position is easily deformed inward so that the wearer easily moves the leg during wearing. On the other hand, a region behind the folding line FL 5 to 15 mm rearward is a region facing the vicinity of the buttocks of the wearer, and by securing the area of the absorbent core 51 in this region, body fluid Can be further decreased.

All of the high rigidity region HR is provided in a region overlapping the constricted region CA as viewed in the width direction W. Since the absorbent core 51 is constricted within the range in which the legs of the wearer are movable, the wearer can easily move the legs. On the other hand, in the area behind the folding line FL, the high rigidity region HR is provided in a region overlapping the constricted region CA as viewed in the width direction W. This makes the absorbent core 51 less collapsible in the width direction W as the absorbent core 51 deviates rearward from the movable range of the legs of the wearer. Therefore, the area of the absorbent core 51 can be provided more reliably as the absorbent core 51 goes rearward. Since the portion behind the absorbent core 51 faces the buttocks of the wearer, the absorbent core 51 is preferably wider in the width direction W than the portion in front of the buttocks. In addition, feces may adhere to the rear portion of the absorbent core 51, but are not absorbed by the absorbent core 51. Preferably, therefore, the area of the absorbent core 51 is provided more reliably, as the absorbent core 51 goes rearward, to decrease the leakage of feces.

Preferably, at least a part of the high rigidity region HR is located behind the rear edges of the pair of grooves 54. The high rigidity region HR is located behind the pair of grooves 54 to allow the absorbent core 51 to be collapsed easily inward in the width direction W. Accordingly, the easy movement of the legs of the wearer in the crotch portion and the provision of the area of the absorbent core 51 on the buttocks side can be achieved.

More preferably, the high rigidity region HR extends rearward from the region overlapping the pair of grooves 54, when viewed in the width direction W, toward the rear edges of the pair of grooves 54. That is, at least a part of the high rigidity region HR may overlap the pair of grooves 54 when viewed in the width direction W. The region where the folding line FL is formed in the crotch region S3 can be collapsed most easily in the width direction W due to the pair of grooves 54. The region where the high rigidity region HR overlaps the pair of grooves 54 when viewed in the width direction W is hardly collapsed in the width direction W, due to the high rigidity region HR, compared to the region where the folding line FL is formed. Further, the portion of the high rigidity region HR extending rearward from the rear edges of the pair of grooves 54 is hardly collapsed in the width direction W due to no presence of the pair of grooves 54. Thus, the absorbent core 51 is gradually less collapsible in the width direction W as the absorbent core 51 goes rearward from the position of the folding line FL of the crotch region S3. This makes it possible to provide a larger area of the absorbent core 51 as the absorbent core 51 goes rearward.

The length of the high rigidity region HR in the width direction W is shorter than the maximum length of the absorbent core 51 in the width direction W. Accordingly, the region outside the high rigidity region HR in the width direction is deformed circularly corresponding to the curvature of the body of the wearer. In other words, the absorbent core 51 is curved in a cup shape in the width direction. Accordingly, the absorbent core 51 is in a shape to accommodate waste matter, such as body fluid, to further decreases the leakage of the body fluid.

The high rigidity region HR is preferably disposed inside the outer edge of the absorbent core 51 in the width direction W, and more preferably inside the outer edge of the narrowest portion LA of the absorbent core 51. The region inside the constricted region CA in the width direction W is subjected to inward force in the width direction W by the force received from both legs of the wearer. Since the high rigidity region HR is provided inside the outer edge of the absorbent core 51 in the width direction W, the absorbent core 51 is hardly deformed inside the width direction W in the high rigidity region HR. This makes it difficult for the absorbent core 51 to collapse on the buttocks side, facilitates provision of the area of the absorbent core 51, and decreases the leakage of the body fluid. Furthermore, if the high rigidity region HR is provided inside the outer edge of the absorbent core 51 in the width direction W, the high rigidity region HR hardly touches the legs of the wearer directly, thus suppressing discomfort feeling to the wearer.

More preferably, the high rigidity region HR is provided in a region between the pair of grooves 54 in the width direction W. In this case, the high rigidity region HR may partially be located on the pair of grooves 54. By providing the high rigidity region HR between the pair of grooves 54 that act as starting points for deforming the absorbent core 51, the absorbent core 51 can be collapsed easily in the width direction in the crotch region S3, and the entire high rigidity region HR is flattened to reliably provide the area of the absorbent core 51.

More preferably, the high rigidity region HR is disposed adjacent to the pair of grooves 54 in the width direction W. This flattens the entire region between the pair of grooves 54 and more reliably provides the area of the absorbent core 51.

As described above, the high rigidity region HR may be formed by the embossed region. Since the emboss region can be provided by embossing to press the absorbent core 51, the high rigidity region HR can be formed without increasing the manufacturing cost of the absorbent article. In addition, the embossed area becomes a region where the absorbent core 51 is hardly collapsed, facilitating the area of the absorbent core 51 more reliably.

Preferably, the embossed region is defined by a region including a plurality of dot-shaped embosses recessed from the skin surface side to the non-skin surface side. When the embosses formed in the embossed region are recessed from the skin surface side to the non-skin surface side, the high rigidity region HR more easily bends in a convex shape toward the non-skin surface side. Therefore, the area of the absorbent core 51 is reliably provided, while the high rigidity region HR is deformed in a smoothly convex shape toward the non-skin surface side according to the curvature of the body shape of the wearer. Accordingly, the absorbent core 51 is in a shape to accommodate waste matter, such as body fluid, and further decreases the leakage of the body fluid.

In the present embodiment, the numerical values relating to the "length" of each part of the absorbent article 10 are measured in the stretched state of the absorbent article 10 stretched until no wrinkles (excluding the folding line FL) are present in the absorbent article 10 when the absorbent article 10 is developed by releasing the side engaging portion 60.

In addition, the rigidity of each part of the absorbent core 51 can be measured by the Gurley bending resistance measurement method in accordance with JIS L-1096 standard (2010). For example, a test piece having a width of 25 mm and a length of 38 mm is first taken from the absorbent core 51 to be measured. Apply cellophane tape to one end of the test piece, that is, a part held by the chuck of the Galley flexing tester. Next, align the lower end of the chuck of the Galley flexing tester with the movable arm scale that matches the dimensions of the test piece. Next, pinch the side of the test piece on which the cellophane tape is applied with the chuck. At this time, the length of the test piece applied to the pendulum of the Galley flexing tester is set to 5 to 6 mm. Next, select the weight of the auxiliary weight and the mounting position so that the reading of the scale of the Galley flexing tester is between 3 and 6, and attach the auxiliary weight. Then, press the switch to rotate the movable arm leftward when the test piece is on the left side of the pendulum, and press the switch to rotate the movable arm rightward when the test piece is on the right side of the pendulum. Then, read the scale at the moment when the rotating rod of the pendulum leaves the test piece. For each test piece, execute the measurement with both right and left rotations of the pendulum.

The bending resistance of the test piece can be calculated by the following equation. Bending resistance (mN) = (25.4 × Wa + 50.8 × Wb + 101.6 × Wc) × (L × 25.4)2 × 3.375 × 10-5 × (RGr + RGl)/(2 × d)
where Wa is the weight (g) of the weight attached to the uppermost attachment position, Wb is the weight (g) of the weight attached to the central attachment position, and Wc is the weight the weight (g) of the weight attached to the lowermost attachment position. L represents the movable arm scale (inch). D represents the width (mm) of the test piece. RGr represents the value of the scale at the moment when the rotating rod of the pendulum is detached from the test piece when the pendulum rotates rightward. RGl represents the value of the scale at the moment when the rotating rod of the pendulum leaves the test piece when the pendulum rotates to the left. The bending resistance of each part of the absorbent core 51 is determined from the bending resistance obtained by the above measurement.

Although the embodiment of the present invention has been described above in detail, it is apparent for persons who have ordinary skill in the art that the embodiment described in this specification does not limit the scope of the present invention. Changes and modifications may apply to the embodiment of the present invention without departing from the scope of the present invention that are defined by the description of the appended claims. The description of the present specification, therefore, has been understood to be illustrative and is in no way intended to limit the scope of the invention.

For example, in the above embodiment, the absorbent body 40 is configured separately from the front waistline member 20 and the rear waistline member 30. Alternatively, the absorbent body 40 may be formed integrally with the front waistline member 20 and the rear waistline member 30.

Further, in the above embodiment, the absorbent article 10 is a so-called pants-type absorbent article having the side engaging portion 60. Alternatively, the absorbent article 10 may be a tape-type absorbent article. In this case, the absorbent article 10 includes no side engaging portion 60, and may include a fastening tape for detachably fixing the front waistline region S1 and the rear waistline region S2 to each other.

According to the above embodiment, the absorbent article capable of reliably providing a space for accommodating feces on the buttocks side during wearing is provided.

According to the present aspect, the absorbent article capable of having a larger absorbing area of body fluid on the buttocks side during wearing may be provided.

### [REFERENCE SIGNS LIST]

- 10: Absorbent article
- 40: Absorbent body
- 50: Absorber
- 51: Absorbent core
- 54: Groove
- CA: Constricted region
- HR: High rigidity region
- S1: Front waistline region
- S2: Rear waistline region
- S3: Crotch region
- FL: Folding line
- L: Front-back direction
- W: Width direction
- T: Thickness direction

## Claims

1. An absorbent article (10), comprising:
a front-back direction (L);
a width direction (W) orthogonal to the front-back direction;
a front waistline region (S1);
a rear waistline region (S2);
a crotch region (S3) disposed between the front waistline region (S1) and the rear waistline region (S2); and
an absorbent core (51) provided at least in the crotch region (S3), wherein
a folding line (FL) for folding the absorbent article in half extends in the width direction in the crotch region (S3),
the absorbent core (51) includes a constricted region (CA) constricted inward in the width direction in the crotch region (S3),
the constricted region (CA) extends in the front-back direction (L) relative to the folding line (FL),
a high rigidity region (HR) is provided behind the folding line (FL) and in a region including the center in the width direction, and
the high rigidity region (HR) is defined by a region having rigidity higher than a rigidity of a region between the folding line (FL) and the high rigidity region (HR),
**characterised in that**:
a length of the high rigidity region (HR) in the front-back direction (L) is longer than the length (L1) between the folding line (FL) and the front edge of the high rigidity region, and
all of the high rigidity region (HR) is provided in a region overlapping the constricted region (CA) when viewed in the width direction (W).

2. The absorbent article according to claim 1, wherein
the front edge of the high rigidity region (HR) is located 5 to 15 mm behind the folding line (FL).

3. The absorbent article according to claim 1 or 2, wherein
the absorbent core (51) includes a constricted region (CA) constricted inward in the width direction in the crotch region (S3), and
the high rigidity region (HR) is provided inside the outer edge of the absorbent core (51) in the width direction.

4. The absorbent article according to any one of claims 1 to 3, wherein
the high rigidity region (HR) has a length in the width direction shorter than a maximum length of the absorbent core (51) in the width direction.

5. The absorbent article according to any one of claims 1 to 4, wherein
the high rigidity region (HR) includes an embossed region in which at least the absorbent core (51) is compressed in the thickness direction (T).

6. The absorbent article according to claim 5, wherein
the embossed region is defined by a region including a plurality of dot-shaped emboss portions recessed from a skin surface side to a non-skin surface side.

7. The absorbent article according to any one of claims 1 to 6, wherein
the absorbent core (51) includes a pair of grooves (54) formed on both sides of a center of the absorbent article (10) in the width direction, and
the pair of grooves (54) extends in the front-back direction (L) relative to the folding line (FL).

8. The absorbent article according to claim 7, wherein
at least a part of the high rigidity region (HR) is located behind the rear edge of the pair of grooves (54).

9. The absorbent article according to claim 7 or 8, wherein
the high rigidity region (HR) extends rearward from a rear edge of the pair of grooves (54) from a region overlapping the pair of grooves when viewed in the width direction.

10. The absorbent article according to any one of claims 7 to 9, wherein
the high rigidity region (HR) is provided in a region between the pair of grooves (54) in the width direction.

11. The absorbent article according to any one of claims 7 to 10, wherein
the high rigidity region (HR) is adjacent to the pair of grooves (54) in the width direction.

## Patentansprüche

1. Absorbierender Artikel (10), der Folgendes umfasst:
eine Vorn-Hinten-Richtung (L);
eine Breitenrichtung (W) rechtwinklig zu der Vorn-Hinten-Richtung;
einen vorderen Taillenbereich (S1);
einen hinteren Taillenbereich (S2);
einen Schrittbereich (S3), der zwischen dem vorderen Taillenbereich (S1) und dem hinteren Taillenbereich (S2) angeordnet ist; und
einen Saugkern (51), der zumindest in dem Schrittbereich (S3) bereitgestellt ist, wobei sich eine Faltlinie (FL) zum Falten des absorbierenden Artikels zur Hälfte in der Breitenrichtung in dem Schrittbereich (S3) erstreckt,
der Saugkern (51) einen verengten Bereich (CA) einschließt, der nach innen in der Breitenrichtung in dem Schrittbereich (S3) verengt ist,
sich der verengte Bereich (CA) in der Vorn-Hinten-Richtung (L) in Bezug auf die Faltlinie (FL) erstreckt,
ein Bereich mit hoher Steifigkeit (HR) hinter der Faltlinie (FL) und in einem Bereich,
der die Mitte in der Breitenrichtung einschließt, bereitgestellt ist und
der Bereich mit hoher Steifigkeit (HR) durch einen Bereich definiert ist, der eine Steifigkeit aufweist, die höher ist als eine Steifigkeit eines Bereichs zwischen der Faltlinie (FL) und dem Bereich mit hoher Steifigkeit (HR),
**dadurch gekennzeichnet, dass**:
eine Länge des Bereichs mit hoher Steifigkeit (HR) in der Vorn-Hinten-Richtung (L) länger ist als die Länge (L1) zwischen der Faltlinie (FL) und dem vorderen Rand des Bereichs mit hoher Steifigkeit und
der gesamte Bereich mit hoher Steifigkeit (HR) in einem Bereich bereitgestellt ist, der mit dem verengten Bereich (CA) bei Betrachtung in der Breitenrichtung (W) überlappt.

2. Absorbierender Artikel nach Anspruch 1, wobei
sich der vordere Rand des Bereichs mit hoher Steifigkeit (HR) 5 bis 15 mm hinter der Faltlinie (FL) befindet.

3. Absorbierender Artikel nach Anspruch 1 oder 2, wobei
der Saugkern (51) einen verengten Bereich (CA) einschließt, der nach innen in der Breitenrichtung in dem Schrittbereich (S3) verengt ist, und
der Bereich mit hoher Steifigkeit (HR) im Inneren vom äußeren Rand des Saugkerns (51) in der Breitenrichtung bereitgestellt ist.

4. Absorbierender Artikel nach einem der Ansprüche 1 bis 3, wobei der Bereich mit hoher Steifigkeit (HR) eine Länge in der Breitenrichtung aufweist, die kürzer ist als eine maximale Länge des Saugkerns (51) in der Breitenrichtung.

5. Absorbierender Artikel nach einem der Ansprüche 1 bis 4, wobei der Bereich mit hoher Steifigkeit (HR) einen geprägten Bereich einschließt, in dem zumindest der Saugkern (51) in der Dickenrichtung (T) komprimiert ist.

6. Absorbierender Artikel nach Anspruch 5, wobei
der geprägte Bereich durch einen Bereich definiert ist, der eine Vielzahl von punktförmigen Prägeteilen einschließt, die von einer Hautoberflächenseite zu einer Nicht-Hautoberflächenseite vertieft sind.

7. Absorbierender Artikel nach einem der Ansprüche 1 bis 6, wobei
der Saugkern (51) ein Paar von Rillen (54) einschließt, die auf beiden Seiten einer Mitte des absorbierenden Artikels (10) in der Breitenrichtung ausgebildet sind, und
sich das Paar von Rillen (54) in der Vorn-Hinten-Richtung (L) in Bezug auf die Faltlinie (FL) erstreckt.

8. Absorbierender Artikel nach Anspruch 7, wobei
sich zumindest ein Teil des Bereichs mit hoher Steifigkeit (HR) hinter dem hinteren Rand des Paars von Rillen (54) befindet.

9. Absorbierender Artikel nach Anspruch 7 oder 8, wobei
sich der Bereich mit hoher Steifigkeit (HR) nach hinten von einem hinteren Rand des Paars von Rillen (54) von einem Bereich, der das Paar von Rillen (54) bei Betrachtung in der Breitenrichtung überlappt, erstreckt.

10. Absorbierender Artikel nach einem der Ansprüche 7 bis 9, wobei der Bereich mit hoher Steifigkeit (HR) in einem Bereich zwischen dem Paar von Rillen (54) in der Breitenrichtung bereitgestellt ist.

11. Absorbierender Artikel nach einem der Ansprüche 7 bis 10, wobei der Bereich mit hoher Steifigkeit (HR) an das Paar von Rillen (54) in der Breitenrichtung angrenzt.

## Revendications

1. Article absorbant (10), comportant :
une direction allant dans le sens avant-arrière (L) ;
une direction allant dans le sens de la largeur (W) orthogonale par rapport à la direction allant dans le sens avant-arrière ;
une région avant au niveau de la taille (S1) ;
une région arrière au niveau de la taille (S2) ;
une région au niveau de l'entrejambe (S3) disposée entre la région avant au niveau de la taille (S1) et la région arrière au niveau de la taille (S2) ; et
une partie centrale absorbante (51) mise en oeuvre au moins dans la région au niveau de l'entrejambe (S3), dans lequel une ligne de pliage (FL) servant à plier l'article absorbant en deux s'étend dans la direction allant dans le sens de la largeur dans la région au niveau de l'entrejambe (S3),
la partie centrale absorbante (51) comprend une région à étranglement (CA) qui est étranglée vers l'intérieur dans la direction allant dans le sens de la largeur dans la région au niveau de l'entrejambe (S3),
la région à étranglement (CA) s'étend dans la direction allant dans le sens avant-arrière (L) par rapport à la ligne de pliage (FL),
une région à rigidité élevée (HR) est mise en oeuvre derrière la ligne de pliage (FL) et dans une région comprenant le centre dans la direction allant dans le sens de la largeur, et
la région à rigidité élevée (HR) est définie par une région ayant une rigidité supérieure à une rigidité d'une région entre la ligne de pliage (FL) et la région à rigidité élevée (HR),
**caractérisé en ce que** :
une longueur de la région à rigidité élevée (HR) dans la direction allant dans le sens avant-arrière (L) est plus longue que la longueur (L1) entre la ligne de pliage (FL) et le bord avant de la région à rigidité élevée, et
la totalité de la région à rigidité élevée (HR) est mise en oeuvre dans une région chevauchant la région à étranglement (CA) lorsque l'on regarde dans la direction allant dans le sens de la largeur (W).

2. Article absorbant selon la revendication 1, dans lequel le bord avant de la région à rigidité élevée (HR) est situé de 5 à 15 mm derrière la ligne de pliage (FL).

3. Article absorbant selon la revendication 1 ou la revendication 2, dans lequel
la partie centrale absorbante (51) comprend une région à étranglement (CA) qui est étranglée vers l'intérieur dans la direction allant dans le sens de la largeur dans la région au niveau de l'entrejambe (S3), et
la région à rigidité élevée (HR) est mise en oeuvre à l'intérieur du bord extérieur de la partie centrale absorbante (51) dans la direction allant dans le sens de la largeur.

4. Article absorbant selon l'une quelconque des revendications 1 à 3, dans lequel
la région à rigidité élevée (HR) a une longueur dans la direction allant dans le sens de la largeur qui est plus courte qu'une longueur maximum de la partie centrale absorbante (51) dans la direction allant dans le sens de la largeur.

5. Article absorbant selon l'une quelconque des revendications 1 à 4, dans lequel
la région à rigidité élevée (HR) comprend une région gaufrée dans laquelle au moins la partie centrale absorbante (51) est comprimée dans la direction allant dans le sens de l'épaisseur (T) .

6. Article absorbant selon la revendication 5, dans lequel la région gaufrée est définie par une région comprenant une pluralité de parties gaufrées en forme de points en retrait depuis un côté orienté vers la surface de la peau jusqu'à un côté non orienté vers la surface de la peau.

7. Article absorbant selon l'une quelconque des revendications 1 à 6, dans lequel
la partie centrale absorbante (51) comprend une paire de rainures (54) formées des deux côtés d'un centre de la partie centrale absorbante (10) dans la direction allant dans le sens de la largeur, et
la paire de rainures (54) s'étend dans la direction allant dans le sens avant-arrière (L) par rapport à la ligne de pliage (FL).

8. Article absorbant selon la revendication 7, dans lequel au moins une partie de la région à rigidité élevée (HR) est située derrière le bord arrière de la paire de rainures (54).

9. Article absorbant selon la revendication 7 ou la revendication 8, dans lequel
la région à rigidité élevée (HR) s'étend vers l'arrière depuis un bord arrière de la paire de rainures (54) depuis une région chevauchant la paire de rainures lorsque l'on regarde dans la direction allant dans le sens de la largeur.

10. Article absorbant selon l'une quelconque des revendications 7 à 9, dans lequel
la région à rigidité élevée (HR) est mise en oeuvre dans une région entre la paire de rainures (54) dans la direction allant dans le sens de la largeur.

11. Article absorbant selon l'une quelconque des revendications 7 à 10, dans lequel
la région à rigidité élevée (HR) est adjacente par rapport à la paire de rainures (54) dans la direction allant dans le sens de la largeur.
